# EUROPÄISCHE PATENTSCHRIFT

(11) **EP 1 220 668 B1**
(45) Veröffentlichungstag und Bekanntmachung des Hinweises auf die Patenterteilung: **15.09.2004**
(21) Anmeldenummer: 99959307.2
(22) Anmeldetag: 23.11.1999
(51) Int. Cl.: A61K 31/155, A61P 33/06

(54) **VERWENDUNG VON DIMINAZEN-DI-ACETURAT UND/ODER PENTAMIDIN ZUR BEHANDLUNG VON MALARIA**
USE OF DIMINAZENE-DI-ACETURATE AND/OR PENTAMIDINE FOR TREATING MALARIA
UTILISATION DE DIMINAZEN-DI-ACETURATE AVEC/OU DE LA PENTAMIDINE POUR TRAITER LA MALARIA

(30) Priorität: 24.11.1998 DE 19853944
(43) Veröffentlichungstag der Anmeldung: 10.07.2002
(73) Patentinhaber: Bourdichon, Alain Jacques, 20095 Hamburg (DE)
(72) Erfinder: Bourdichon, Alain Jacques, 20095 Hamburg (DE)
(74) Vertreter: Schneider, Henry, Dipl.-Ing.
(86) Internationale Anmeldenummer: PCT/EP1999/009026
(87) Internationale Veröffentlichungsnummer: WO 2000/030631

(56) Entgegenhaltungen:
- EP-A- 0 366 066
- HEISCHKEIL R.: "Wirksamkeit der Trypanozide Berenil und Pentamidin bei der Nagetiermalaria (Plasmodium vinckei)" ZEITSCHRIFT FÜR TROPENMEDIZIN UND PARASITOLOGIE, Bd. 22, Nr. 3, September 1971 (1971-09), Seiten 243-249, XP000885855
- WALZER P. D., ET AL.: "Cationic antitrypanosomal and other antimicrobial agents in the therapy of experimental Pneumocystis-carinii pneumonia" ANTIMICROB AGENTS CHEMOTHER., Bd. 32, Nr. 6, 1988, Seiten 896-905, XP000885981

## Beschreibung

Die Erfindung betrifft die Verwendung von Diminazen-di-aceturat und/oder Pentamidin zur Herstellung von Arzneimitteln zur Behandlung von Malaria sowie ein pharmazeutisches Präparat zur Behandlung von Malaria und Trypanosomiasis, das Diminazen-di-aceturat und Procain oder ein Procain-Derivat enthält.

In Ländern mit tropischem Klima sind Infektionskrankheiten, die durch Protozoen hervorgerufen werden, weit verbreitet, wie z.B. Malaria und die sogenannte Schlafkrankheit (Trypanosomiasis). Von der Malaria sind je nach Erreger verschiedene Formen bekannt, die teilweise nach kurzer Zeit mit hoher Wahrscheinlichkeit zum Tode führen. Die Behandlung beider Krankheiten wird chemotherapeutisch durchgeführt.

Gegen die Trypanosomiasis sind Chemotherapeutika auf Diminazen-Basis bekannt, die in Pulverform erhältlich sind, so dass die Injektionslösung vor der Injektion noch gemischt werden muss, oder die bereits als wässrige Lösung vorliegen, wobei mitunter Lösungsvermittler verwendet werden, um stabile Lösungen zu erhalten. Diminazen-di-aceturathaltige Präparate werden auch mit Procain oder Lidocain kombiniert, um Schmerzzustände bei der Injektion zu unterdrücken.

Zur Behandlung der Malaria werden verschiedene natürliche und synthetische Stoffe, wie Chinaalkoloide, Chloroquni u. dgl. Verwendet.

Zunehmend problematisch sind Resistenzen der Erreger, insbesondere bei der Malaria, so dass ständig Bedarf an neuen wirksamen Medikamenten besteht.

Es ist daher Aufgabe der Erfindung, ein Präparat zu schaffen, das zur Behandlung der Malaria verwendet werden kann.

Diese Aufgabe wird durch die Merkmale des Anspruchs 1 gelöst.

Dazu ist erfindungsgemäß vorgesehen, Diminazen-di-aceturat und oder Pentamidin zur Herstellung eines Arzneimittels zur Behandlung der Malaria zu verwenden, wobei Procain oder ein Procainderivat und/oder Lidocain oder ein Lidocainderivat in Kombination mit den vorgenannten Wirkstoffen verwendet werden, da durch die Kombination die Wirkung derart verstärkt wird, dass die Erreger sicher abgetötet werden.

Die Verwendung von Diminazen-di-aceturat und Pentamidin ist zur Behandlung der Trypanosomiasis bereits bekannt, jedoch hat die erfindungsgemäße Verwendung zur Behandlung der Malaria zu nicht vorhersehbaren Erfolgen geführt. Zudem sind dem Stand der Technik keinerlei Hinweise zu entnehmen, diese Stoffe erfindungsgemäß zu verwenden. Es war zudem auch nicht zu Erwarten, daß die erfindungsgemäße Verwendung den gewünschten Erfolg zeigt, da sich der Reproduktionszyklus der Malaria-Erreger, die als Sporozoiten, Schizonten, Merozoiten und Gameten vorliegen, sich von dem anderer Protozoen unterscheidet. Diminazen-di-aceturat ist vorteilhafterweise in einer Konzentration von nur 1 µM bereits in Plasmodien-Kulturen wirksam.

Da bei Protozoeninfektionen stets die Gefahr von Sekundärinfektionen durch andere Erreger, wie Bakterien o. dgl. aufgrund der naturgemäß stark geschwächten Abwehrkräfte besteht, ist nach einer bevorzugten Ausführungsform ein erfindungsgemäßes pharmazeutisches Präparat zur Behandlung der Malaria und auch der Trypanomiasis vorgesehen, das Diminazen-di-aceturat und Procain oder ein Procainderivat und/oder Lidocain oder ein Lidocaiderivat sowie einen antibiotischen Wirkstoff enthält. Neben Diminazen-di-aceturat oder auch statt dessen kann hier als Wirkstoff auch Pentamidin verwendet werden. Besonders vorteilhaft ist die Kombination von Diminazen-di-aceturat und Pentamidin, da diese teilweise ein unterschiedliches Wirkungsspektrum aufweisen und sich somit in ihrer Wirkung ergänzen, so daß nahezu alle Arten von Blutparasiten erfaßt werden. Zudem werden durch die Kombination vorteilhafterweise Resistenzen vermieden.

Als antibiotischer Wirkstoff wird vorzugsweise Penicillin oder ein Penicillin-Derivat verwendet. Vorteilhaft ist hier insbesondere die Verwendung in Form von Procain-Benzylpenicillinat, da dadurch die Wirkdauer des Penicillins verlängert wird. Auch der Einsatz von Tetracyclin ist erfindungsgemäß vorgesehen, wobei hier zusätzlich noch die Wirksamkeit gegenüber der Anaplosmosis gegeben ist.

Es können aber auch andere antibiotisch wirkende Stoffe, wie z. B. Nisin eingesetzt werden.

Nach einer bevorzugten Ausführungsform enthält das Präparat weiterhin Phenazon, das als Analgetikum wirkt, wodurch Schmerz- und Fieberzustände, die durch die Injektion bzw. die Infektion bedingt sind und die bei Protozoeninfektionen wie Malaria und Trypanomiasis auftreten, gelindert werden.

Das Procain oder Lidocain, das anstelle von Procain oder gemeinsam mit diesem verwendet werden kann, und die entsprechenden Derivate dienen dazu, das mit der Protozoeninfektion auftretende hohe Fieber abzusenken und die ggf. mit der Injektion verbundenen starken Schmerzen zu vermeiden, die zu tödlichen Schockzuständen führen können. Von wesentlich größerer Bedeutung, wie oben beschrieben, ist jedoch eine verstärkende Wirkung auf die bioziden Wirkstoffe aus der Gruppe der Amidine, d. h. Diminazen-di-aceturat und Pentamidin, da dadurch eine sichere Abtötung der Protozoen, insbesondere der Malaria-Erreger gewährleistet ist.

Als Fieber- und Schmerzmittel kann zusätzlich auch Acetylsalicylsäure oder ein Derivat verwendet werden.

Nach einer weiteren Ausführungsform beinhaltet das erfindungsgemäße Präparat Isometadium-Hydrochlorid aus der Gruppe der Phenathridine oder Nitronidazol als weiteren bioziden Wirkstoff, die zusätzlich oder alternativ zum Pentamidin und/oder Diminazen-di-aceturat im Präparat enthalten sein können.

Das erfindungsgemäße Präparat kann in verschiedenen Darreichungsformen vorliegen. So kann das Präparat in Tablettenform, als wäßrige Lösung zur Injektion, ggf. unter Verwendung von Lösungsvermittlern, als Suspension ebenfalls zur Injektion, auch als Suspension von Lipidvesikeln (Liposomen) oder in anderer geeigneter Weise vorliegen.

Das erfindungsgemäße Präparat, das im human- und veterinärmedizinschen Bereich einsetzbar ist, ist zumindest gegenüber folgenden Protozoen wirksam:
- Trypanosomes congolense, Tryp. vivax, Tryp. Bruccei und T. Evansi
- Piroplasmose (Piroplasma motasi, Piroplasma caballi) Babesiose (Babesia bigemina, Babesia motasi, Babesia canis und andere Babesien)
- Theileria annulata
- Plasmodien (Plasmodium vivax, Plasmodium malaria, Plasmodium falciparum, Plasmodium ovalae).

Weitere vorteilhafte Ausgestaltungen werden in den Unteransprüchen gekennzeichnet.

## Patentansprüche

1. Verwendung von Diminazen-di-aceturat und/oder Pentamidin zur Herstellung von Arzneimitteln zur Behandlung von Malaria, wobei Diminazen-di-aceturat und/oder Pentamidin mit Procain oder einem Procainderivat und/oder Lidocain oder einem Lidocainderivat kombiniert ist.

2. Präparat zur Behandlung von Malaria und Trypanosomiasis, das Diminazen-di-aceturat und Procain oder ein Procain-Derivat enthält,
**dadurch gekennzeichnet, dass**
das Präparat einen antibiotischen Wirkstoff enthält.

3. Pharmazeutisches Präparat nach Anspruch 2,
**dadurch gekennzeichnet, dass**
der antibiotische Wirkstoff Penicillin oder ein Penicillin-Derivat ist.

4. Pharmazeutisches Präparat nach Anspruch 3,
dad urch gekennzeichnet, dass
das Präparat Pocain-Benzylpenicillinat enthält.

5. Pharmazeutisches Präparat nach Anspruch 2,
**dadurch gekennzeichnet, dass**
der antibiotische Wirkstoff ein Tetracyclin ist.

6. Pharmazeutisches Präparat nach Anspruch 2,
**dadurch gekennzeichnet, dass**
der antibiotische Wirkstoff Nisin ist.

7. Pharmazeutisches Präparat nach einem der Ansprüche 2 bis 6,
**dadurch gekennzeichnet, dass**
statt Procain oder eines Procain-Derivates Lidocain oder ein Lidocain-Derivat im Präparat enthalten ist.

8. Pharmazeutisches Präparat nach einem der Ansprüche 2 bis 6,
**dadurch gekennzeichnet, dass**
neben Procain oder dem Procain-Derivat Lidocain oder ein Lidocain-Derivat im Präparat enthalten ist.

9. Pharmazeutisches Präparat nach einem der Ansprüche 2 bis 8,
**dadurch gekennzeichnet, dass**
statt Diminazen-di-aceturat Pentamidin und/oder Nitronidazol und/oder Isometadium-Hydrochlorid im Präparat enthalten ist.

10. Pharmazeutisches Präparat nach einem der Ansprüche 2 bis 8,
**dadurch gekennzeichnet, dass**
neben Diminazen-di-aceturat Pentamidin und/oder Nitronidazol . und/oder Isometadium-Hydrochlorid im Präparat enthalten ist.

11. Pharmazeutisches Präparat nach einem der Ansprüche 2 bis 10,
**dadurch gekennzeichnet, dass**
das Präparat in Tablettenform vorliegt.

12. Pharmazeutisches Präparat nach einem der Ansprüche 2 bis 10,
**dadurch gekennzeichnet, dass**
das Präparat als wässrige Lösung vorliegt, wobei ein Lösungsvermittler in der wässrigen Lösung enthalten sein kann.

13. Pharmazeutisches Präparat nach Anspruch 12,
**dadurch gekennzeichnet, dass**
als Lösungsvermittler eine Celluloseether und ggf. Glycerin in der wässrigen Lösung enthalten ist.

14. Pharmazeutisches Präparat nach einem der Ansprüche 2 bis 10,
**dadurch gekennzeichnet, dass**
das Präparat in Form einer Suspension, auch in Form einer Suspension von Lipidvesikeln, die die Wirkstoffe einschließen, vorliegt.

15. Pharmazeutisches Präparat nach einem der Ansprüche 2 bis 14,
**dadurch gekennzeichnet, dass**
das Präparat Phenazon und/oder Acetylsalicylsäure bzw. ein entsprechendes Derivat enthält.

## Claims

1. Use of diminazene di aceturate and/or pentamidine for the manufacture of pharmaceuticals for treatment of malaria, wherein diminazene di aceturate and/or pentamidine is combined with procaine or a procaine derivative and/or lidocaine or a lidocaine derivative.

2. A preparation for treatment of malaria and trypanosomiasis, containing diminazene di aceturate and procaine or a procaine derivative,
**characterised in that**
the preparation contains an antibiotic active ingredient.

3. A pharmaceutical preparation according to claim 2,
**characterised in that**
the antibiotic active ingredient is penicillin or a penicillin derivative.

4. A pharmaceutical preparation according to claim 3,
**characterised in that**
the preparation contains procaine benzylpenicillinate.

5. A pharmaceutical preparation according to claim 2,
**characterised in that**
the antibiotic active ingredient is a tetracycline.

6. A pharmaceutical preparation according to claim 2,
**characterised in that**
the antibiotic active ingredient is nisin.

7. A pharmaceutical preparation according to any of claims 2 to 6,
**characterised in that**
the preparation contains lidocaine or a lidocaine derivative instead of procaine or a procaine derivative.

8. A pharmaceutical preparation according to any of claims 2 to 6,
**characterised in that**
the preparation contains lidocaine or a lidocaine derivative in addition to procaine or a procaine derivative.

9. A pharmaceutical preparation according to any of claims 2 to 8,
**characterised in that**
the preparation contains pentamidine and/or nitronidazole and/or isometamidium hydrochloride instead of diminazene di aceturate.

10. A pharmaceutical preparation according to any of claims 2 to 8,
**characterised in that**
the preparation contains pentamidine and/or nitronidazole and/or isometamidium hydrochloride in addition to diminazene di aceturate.

11. A pharmaceutical preparation according to any of claims 2 to 10,
**characterised in that**
the preparation takes the form of tablets.

12. A pharmaceutical preparation according to any of claims 2 to 10,
**characterised in that**
the preparation takes the form of an aqueous solution, wherein the aqueous solution may contain a detergent.

13. A pharmaceutical preparation according to claim 12,
**characterised in that**
the aqueous solution contains a cellulose ether and possibly glycerin as a detergent.

14. A pharmaceutical preparation according to any of claims 2 to 10,
**characterised in that**
the preparation takes the form of a suspension or of a suspension of lipid vesicles, which contain the active ingredients.

15. A pharmaceutical preparation according to any of claims 2 to 14,
**characterised in that**
the preparation contains phenazone and/or acetylsalicylic acid or a corresponding derivative.

## Revendications

1. Utilisation de diminazène-di-aceturat et/ou de pentamidine pour la fabrication de médicaments pour le traitement de la malaria, dans laquelle diminazène-di-aceturat et/ou pentamidine est/sont combiné(s) à de la procaïne ou un dérivé de procaïne et/ou de la lidocaïne ou un dérivé de lidocaïne.

2. Préparation pour le traitement de la malaria et de la trypanosomiase, contenant du diminazène-di-aceturat et de la procaïne ou un dérivé de procaïne, et
**caractérisée en ce que**
la préparation contient un agent actif antibiotique.

3. Préparation pharmaceutique selon la revendication 2,
**caractérisée en ce que**
l'agent actif antibiotique est de la pénicilline ou un dérivé de pénicilline.

4. Préparation pharmaceutique selon la revendication 3,
**caractérisée en ce que**
la préparation contient de la procaïne-benzylpenicillinate .

5. Préparation pharmaceutique selon la revendication 2,
**caractérisée en ce que**
l'agent actif antibiotique est une tétracycline.

6. Préparation pharmaceutique selon la revendication 2,
**caractérisée en ce que**
l'agent actif antibiotique est de la nisine.

7. Préparation pharmaceutique selon l'une quelconque des revendications 2 à 6,
**caractérisée en ce que**
la préparation contient de la lidocaïne ou un dérivé de lidocaïne au lieu de la procaïne ou d'un dérivé de procaïne.

8. Préparation pharmaceutique selon l'une quelconque des revendications 2 à 6,
**caractérisée en ce que**
la préparation contient de la lidocaïne ou un dérivé de lidocaïne aussi bien que de la procaïne ou un dérivé de procaïne.

9. Préparation pharmaceutique selon l'une quelconque des revendications 2 à 8,
**caractérisée en ce que**
la préparation contient de la pentamidine et/ou du nitronidazol et/ou de l'isometamidium-hydrochlorid au lieu du diminazène-di-aceturat.

10. Préparation pharmaceutique selon l'une quelconque des revendications 2 à 8,
**caractérisée en ce que**
la préparation contient de la pentamidine et/ou du nitronidazole et/ou de l'isometamidium-hydrochlorid aussi bien que du diminazène-di-aceturat.

11. Préparation pharmaceutique selon l'une quelconque des revendications 2 à 10,
**caractérisée en ce que**
la préparation est sous la forme de tablettes.

12. Préparation pharmaceutique selon l'une quelconque des revendications 2 à 10,
**caractérisée en ce que**
la préparation est sous la forme d'une solution aqueuse, laquelle peut contenir un détergent.

13. Préparation pharmaceutique selon la revendication 12,
**caractérisée en ce que**
la solution aqueuse contient un éther de cellulose et, le cas échéant, de la glycerine.

14. Préparation pharmaceutique selon l'une quelconque des revendications 2 à 10,
**caractérisée en ce que**
la préparation prend la forme d'une suspension ou d'une suspension de particules lipidiques, qui renferment les agents actifs.

15. Préparation pharmaceutique selon l'une quelconque des revendications 2 à 14,
**caractérisée en ce que**
la préparation contient du phénazone et/ou de l'acide acétylsalicylique ou un dérivé correspondant.
